# EUROPEAN PATENT APPLICATION

(11) **EP 3 769 837 A1**
(43) Date of publication of application: **27.01.2021**
(21) Application number: 19382616.1
(22) Date of filing: 22.07.2019
(51) Int. Cl.: B01J 23/80, B01J 37/03, B01J 37/06, B01J 37/08, B01J 35/00, B01J 37/00, C07C 29/154

(54) **CO-PRECIPITATION METHOD AND CATALYST THEREOF**

(71) Applicant: Fundació Institut Català d'Investigació Química (ICIQ), 43007 Tarragona (ES)
(72) Inventor: Urakawa, Atsushi, Kasuga, Fukuoka 816-0813 (JP); Phongprueksathat, Nat, 84000 Surat Thani (TH)

(57) **Abstract**

The present invention relates to a method for the preparation of a catalyst precursor comprising the steps of (i) co-precipitating a mixture of metal salts comprising copper acetate in the presence of urea to obtain a solid, (ii) separating the solid obtained in step (i), and (iii) calcinating the solid obtained in step (ii). The present invention also relates to a catalyst obtained from said catalyst precursor and compositions comprising said catalyst or said catalyst precursor, as well as their use in methanol synthesis or in the water gas shift reaction.

## Description

The present invention relates to a method for the preparation of a catalyst precursor or a catalyst comprising the steps of (i) co-precipitating a mixture of metal salts comprising copper acetate in the presence of urea to obtain a solid, (ii) separating the solid obtained in step (i), and (iii) calcinating the solid obtained in step (ii). The present invention also relates to a catalyst precursor, a catalyst, a precursor catalyst composition and a catalyst composition obtained by means of the methods of the present invention and their use in methanol synthesis or in the low temperature water gas shift reaction.

### BACKGROUND ART

Most industrial chemical processes rely on the use of solid catalysts that interact with liquids or gasses and promote the desired chemical transformation. This is the case for instance of cracking and reforming processes, the Haber-Bosch process, syngas preparation and syngas transformation to fuels or other industrially relevant building blocks, among other processes. In such processes, the efficiency of the catalyst is mostly associated to its specific surface area accessible to accommodate reactive substrates (for example, hydrogen molecules) at suitable sites to promote the desired transformation. There is consequently a strong relationship between the structure of the catalyst at the microscopic and nanoscopic scales and its catalytic performance.

In catalytic systems comprising several solid phases, such as binary or ternary catalyst, the spatial arrangement of atoms in the catalyst structure is of utmost importance since several solid phases (or different metals) often need to interact to allow the reaction to take place. In addition, certain elements are often added in order to prevent the reactive site from deactivation. This is, for instance, the case of copper-based catalysts useful in the transformation of carbon oxides (this is, CO or CO₂) that typically tend to form unreactive aggregates by sintering. To avoid the sintering of copper reactive sites, a common strategy consists in preparing a binary system through the addition of an additional metallic species, such as zinc oxide, in order to isolate the reactive sites one from each other and, thus, avoid sintering. As a consequence, chemists are constantly developing new methods for the preparation of solid catalysts in order to control the way atoms are spatially arranged at the microscopic level and optimize catalyst efficiency and stability.

Several methods are known in the art for the preparation of metal-based solid catalysts, as summarized by Hutchings and co-workers in Basic Principles in Applied Catalysis ("Heterogeneous Catalyst preparation" pp.215-258, Springer Science & Business Media, 2013, ISBN 3662059819), including, for example, the co-precipitation method, the impregnation method and the sol-gel method.

A method of manufacturing a catalyst typically comprises the steps of: (i) precipitation of solids, for instance, of hydroxide metal salts (by, for example, sol-gel methods, co-precipitation, hydrolysis and deposition), (ii) hydrothermal transformation, (iii) separation of solids (by means of, for example, decantation, filtration and centrifugation), (iv) washing, (v) crushing and grinding of the solid, (vi), shaping of the catalyst, (vii) calcination, (viii) impregnation, (ix) mixing and (x) activation. In the particular case of bulk catalysts prepared by the precipitation method, several factors have been identified that can influence the nature of the precipitate and, consequently, the efficiency of the catalyst. These factors are, for example, solution composition (for example, purity and metal salts), pH of the solution, aging (for example, time and temperature), additives, precipitating agent (for example, base), concentration, solvent, temperature and mixing sequence.

In the specific case of the co-precipitation method, different metal hydroxide salts are precipitated together from a basic solution that is most commonly maintained at a constant pH (buffer solution). Methanol synthesis catalysts or catalysts for the low temperature waster gas shift reaction, based on copper (Cu), zinc oxide (ZnO) and, eventually, aluminium oxide (Al₂O₃) are typically prepared through this general method. Different factors are known to affect the structure of the precipitate and catalyst efficiency:
- In the first place, the base must be carefully chosen as the associated cation will likely interact with the precipitate. In the case of Cu/ZnO/Al₂O₃ catalysts, sodium carbonate is most commonly used as a base. In such cases, a washing of the prepared precipitate is essential to remove sodium cations adsorbed on the surface of the catalyst precursor (the presence of sodium cations in the catalyst leads to poor catalytic activity). The base also needs to be used in sufficient amounts to allow for a pH of the solution sufficient for the precipitation of the metal salts to take place.
- The pH value must be carefully selected so that it allows for the precipitation of the desired species, taking into account that the cation of the metal hydroxide generally determines the precipitation pH. For copper-zinc binary systems prepared by co-precipitation, a suitable pH value for the precipitation is typically above about 7.

- The amount ratio of metal cations engaged in the process also influences significantly the efficiency of the catalyst. As the different cations do not precipitate as hydroxide salts at the same pH values, the ratio of cations in the precipitate is not always the same as the ratio of cations engaged in the co-precipitation method. This ratio has some influences on catalyst efficiency as well, as binary systems are often used to combine reactivity and stability brought by different metal cations; in the case of copper-zinc systems, copper is the active metal and zinc oxide is a stabilizing agent preventing copper active sites from sintering.
- Finally, another factor having substantial influence on the efficiency of the catalyst is the choice of the metal salt engaged in the co-precipitation process. The anions present in the metal salt can indeed act as catalyst poisoning agents. This is, for instance, the case of chlorides and sulphate for copper-based catalysts. Quite commonly, nitrate salts are used as metal salt precursors, as the nitrate adsorbed on the surface of the catalyst is removed during the washing step and/or during the calcination step. This has the disadvantage of generating some contamination waste, either in the form of nitrate containing wastewater (from the washing step) or in the form of emission of NOₓ gases (known for their potent greenhouse effects).

Atake et al. have reported in 2007 (Journal of Molecular Catalysis A: Chemical 275 (2007) 130-138) the synthesis via homogeneous precipitation of a copper-based catalyst precursor for the water gas shift reaction (CO + H₂O → CO₂ + H₂). The employed procedure consists in mixing nitrate salts of copper, zinc and, optionally, aluminium, in an aqueous solution of urea (in large excess). The use of urea as a base in the co-precipitation procedure is advantageous as urea is progressively hydrolysed upon heating (80-90 °C) and releases hydroxide ions, according to the equation:

CO(NH₂)₂ + 3 H₂O → 2 NH₄⁺ + HCO₃⁻ + OH⁻

This allows gently modulating the pH of the solution and controlling the precipitation process through urea addition. Under these conditions, metal nitrates salts are converted to metal hydroxides and then to metal hydroxycarbonates which form the catalyst precursor. Such method allows to prepare a catalyst precursor comprising a crystalline phase of aurichalcite (Cu,Zn)₅(CO₃)₂(OH)₆, which appears to be a precursor of the active catalyst for the water gas shift reaction. According to the authors, the addition of other elements, such as aluminium, together with variations of the aging time do affect the crystalline structure of the catalyst precursor and its activity.

Fan and co-workers (Fuel Processing Technology 167 (2017) 69-77) have used the homogenous precipitation method in the preparation of a methanol synthesis catalyst based on copper and zinc oxide. According to the authors, the efficiency of the catalyst is related to particle size, surface area, metallic surface area and composition of the catalyst, which can be controlled through the preparation process of the catalyst. The authors proposed to use a homogeneous precipitation method induced by urea hydrolysis using copper and zinc nitrate salts, to prepare an efficient catalyst for methanol synthesis from syngas. In the reported preparation process, the molar ratio of Cu to Zn salts in the prepared catalyst is 1:1, the precipitation step is carried out at a temperature in the range of 70 to 95 °C by simultaneous dilution of solutions of urea on the one hand and metal salts on the other hand (simultaneous addition of solutions via syringe pumps in water). The precipitation step is followed by an aging step, a filtration step, a washing step and a drying step prior to the final calcination step giving rise to the catalyst precursor. Before the calcination step, the catalyst comprises principally aurichalcite crystalline phase. The prepared catalyst, once activated, exhibits slightly improved catalytic activity (i.e. higher conversion) in low temperature methanol synthesis from syngas over the catalyst prepared by a more conventional co-precipitation method based on the precipitation of copper and nitrate salts induced by sodium carbonate as a base.

In an attempt to avoid the generation of contaminating nitrate containing wastewater in the co-precipitation process leading to the formation of Cu/ZnO catalysts of methanol synthesis, Behrens et al. (Chem. Commun., 2011, 47, 1701-1703) reported mixed basic formate of copper and zinc, of general formula (Cu₁₋ₓZnₓ)₂(OH)₃HCO₂, as promising candidates for catalyst precursors. Such mixed basic formates of copper and zinc can be generated in situ from Cu₂(OH)₂CO₃ and ZnO in a solution of formic acid in the presence of sodium carbonate, leading to suitable catalysts precursors with moderate activity. The reported preparation method comprises a washing step, useful to remove sodium cations coming from the base. Authors also highlight the relevance of the preparation process of a catalyst in the determination of the meso and nanostructuring of the catalyst, allowing for a high dispersion of the copper active phase as well as an intimate contact between copper and zinc oxide.

Prieto and co-workers (Catalysis Today 215 (2013) 142- 151) have also reported some intents to optimize the preparation process of Cu/ZnO methanol synthesis catalysts through the replacement of the traditionally used sodium carbonate base by ammonium hydrogencarbonate (NH₄HCO₃). Solutions of nitrate salts of copper and zinc were progressively added via a syringe pump to water together with a solution of either sodium carbonate or NH₄HCO₃ during the precipitation step. After aging (2 hours) and filtration, the isolated solid was either directly calcined or submitted to several washing steps with deionized water (in order to remove sodium and ammonium ions trapped in the catalyst precursor). In both cases, the resulting crystalline phase in the catalyst precursor is zincian malachite. The authors report that the washing steps have a positive impact on catalyst activity in both cases, which is explained through an adequate crystallite size of CuO in the calcined catalyst precursor enabled by the removal of trapped cations on the surface of copper. The washing step is thus reported to be necessary to obtain optimal activity.

Smith et al. have reported a method of preparation of a Cu/ZnO methanol synthesis catalyst derived from an amorphous zincian georgite precursor (Chem. Sci., 2017, 8, 2436). According to the authors, the described catalyst is also useful in the low temperature water gas shift reaction. The developed method comprises the step of precipitating a zincian georgite phase by heating at 40 °C a solution of acetate salts of copper and zinc (2:1 ratio) containing a base (ammonium carbonate, ammonium hydrogencarbonate or sodium carbonate). A short aging period of 15 minutes is used in order to favour the formation of the amorphous precipitate. Obtained precipitates were filtered and washed with deionized water prior to calcination. Resulting catalysts were tested in methanol synthesis from syngas and proved to be less efficient than the standard methanol synthesis commercial catalyst. Also, authors indicate that the washing step is beneficial in providing an efficient catalyst (unwashing of the catalyst is described to favour sintering of the metal oxides crystallites by decomposition of a carbonate-rich phase at high temperature). This method is reported not to produce aurichalcite phase in the catalyst precursor, nor any other crystalline phase. Authors also point out that zincian malachite is the preferred crystalline phase for the preparation of precursors of catalysts for methanol synthesis.

In relation to methanol synthesis, Bansode and co-workers (Journal of Catalysis 343 (2016) 127-132) reported a process facilitating the hydrogenation of carbon dioxide at high pressure wherein a mixture of carbon dioxide and hydrogen (1:>3 ratio) is passed through a reactor filled with a commercial methanol synthesis catalyst. Such process is typically carried out pressures of at least 200 bar and temperatures of 220-300 °C. In ideal conditions, the reported process allows converting up to 90% carbon dioxide into methanol with a selectivity of 95% in one sole-pass through the reactor using the commercial catalyst, with a weight time yield of up to 2.4 grams of methanol per gram of catalyst per hour.

From what is known in the art, it derives that there is still a need for providing improved methods for the preparation of efficient copper-based heterogeneous catalysts that generate less-contaminating wastewater and/or that do not require a washing step.

### SUMMARY OF THE INVENTION

The inventors after extensive and exhaustive research have developed a new method for the preparation of a copper-based catalyst precursor or catalyst based on the homogeneous precipitation of a mixture of metal salts comprising copper acetate in the presence of urea. The inventors have found that this method allows preparing a crystalline or partially crystalline catalyst precursor that can advantageously and surprisingly be used in catalytic processes without a need for its preparation to involve a washing step, or wherein said preparation provides a reduced amount of nitrate anions in the generated wastewater. The developed method thus generates a less contaminating wastewater and allows preparing a catalyst in a more efficient manner, since the step of washing, reported in the prior art to be beneficial to catalyst efficiency, may either be omitted, or may generate a less-contaminating wastewater, with no or negligible effect on catalyst efficiency, unlike the methods described in the state of the art. According to the methods described in the state of the art, when salts other than copper (II) nitrate salts are used in the preparation of a copper-based methanol synthesis catalyst precursor, the efficiency of the resulting catalyst is typically lower than for catalysts prepared from copper (II) nitrate salts. The invention thus provides an improved preparation method for the preparation of a copper-based catalyst which, without using salts of copper (II) nitrate, surprisingly exhibits an efficiency that is similar or comparable to the efficiency of catalysts prepared using copper (II) nitrate as metal precursor, while presenting the further advantage of generating a less contaminating wastewater, or no wastewater at all. The invention thus provides an improved method for the preparation of a copper-based catalyst wherein the method presents both a reduced need for waste management and a reduced environmental impact, said method allowing preserving the level of activity and efficiency of the catalyst.

The inventors have in particular developed a method for the preparation of a methanol synthesis catalyst, which produces a crystalline catalyst precursor (aurichalcite phase) and provides an efficient catalyst without using nitrate salts of copper (II), starting from a mixture of copper acetate with zinc acetate (preferably, a mixture 1:1 of copper:zinc). The resulting catalyst precursor, once activated, is highly efficient in the conversion of carbon dioxide to methanol by high-pressure CO₂ hydrogenation and even surpasses the activity of standard commercial methanol synthesis catalysts based on Cu/ZnO/Al₂O₃. Such catalyst may also be useful in the low temperature water gas shift reaction, this one being part of the equilibria involved in the methanol synthesis.

Thus, in a first aspect, the present invention relates to a method for the preparation of a catalyst precursor comprising the steps of:
(i) heating an aqueous solution of urea with a mixture of metal salts comprising at least a compound of formula Cu(OCOCH₃)₂, a solvate thereof or a co-crystal thereof, to obtain a solid, and wherein the molar amount of urea is at least three times the total molar amount of metal salts;
(ii) separating the solid obtained in step (i) from the aqueous solution;
(iii) calcinating the solid obtained in step (ii).

In a second aspect, the present invention relates to a catalyst precursor obtained by means of a method for the preparation of a catalyst precursor of the present invention.

In a third aspect, the present invention refers to a method for the preparation of a catalyst comprising the step of activating the catalyst precursor of the present invention.

In a fourth aspect, the present invention relates to a catalyst obtained by means of activation of a catalyst precursor of the present invention. According to the present invention, the resulting catalyst is particularly useful as methanol synthesis catalyst from carbon dioxide and hydrogen. It may also be useful as a catalyst in the low temperature water gas shift reaction.

In a fifth aspect, the present invention refers to a composition comprising a catalyst (catalyst composition) or a catalyst precursor (catalyst precursor composition) of the present invention (this is, a catalyst or a catalyst precursor obtained by means of a method of the present invention).

In a sixth aspect, the present invention thus relates to the use of the catalyst of the present invention or a composition comprising a catalyst of the present invention as a catalyst for the preparation of methanol by hydrogenation of carbon dioxide or for the low temperature water gas sift reaction.

In a seventh and final aspect, the present invention refers to method for the preparation of methanol by hydrogenation of carbon dioxide comprising the step of contacting carbon dioxide with hydrogen in the presence of a catalytically effective amount of a catalyst or a catalyst composition, both of the present invention.

### BRIEF DESCRIPTION OF THE DRAWINGS

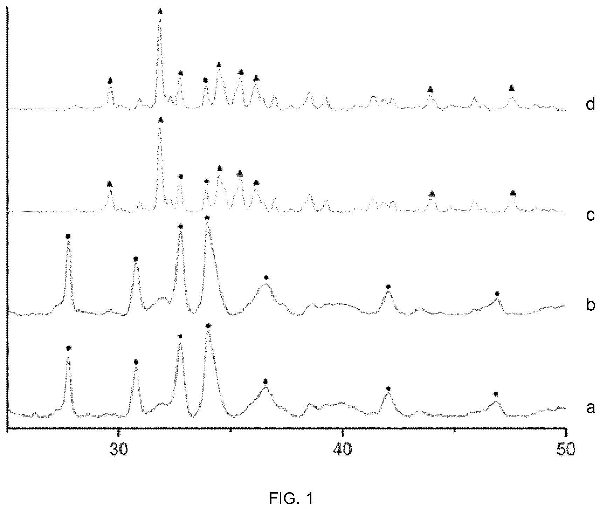

Fig. 1 shows the X-ray powder diffraction pattern (2θ in degrees as x axis and peak intensity as y axis) of a) the catalyst precursor after homogenous precipitation of a 1:1 mixture of copper acetate and zinc acetate in urea after filtration and washing with deionized water; b) the catalyst precursor after homogenous precipitation of a 1:1 mixture of copper acetate and zinc acetate in urea after filtration with no washing step; c) the catalyst precursor after homogenous precipitation of a 1:1 mixture of copper nitrate and zinc nitrate in urea after filtration and washing with deionized water; d) the catalyst precursor after homogenous precipitation of a 1:1 mixture of copper nitrate and zinc nitrate in urea after filtration with no washing step; the black triangles indicate the presence of a rosasite crystalline phase while the black circles indicate the presence of aurichalcite crystalline phase.

### DETAILED DESCRIPTION OF THE INVENTION

All terms as used herein in this application, unless otherwise stated, shall be understood in their ordinary meaning as known in the state of the art. Other more specific definitions for certain terms as used in the present application are as set forth below and are intended to apply uniformly throughout the specification and claims, unless an otherwise expressly set out definition provides a broader definition.

For the purposes of the present invention, any ranges given include both the lower and the upper end-points of the range. Ranges given, such as temperatures, times, molar ratio and the like, should be considered approximate (this is, with a 5% margin of variation around indicated point), unless specifically stated otherwise.

In the context of the present invention, the term "metal salt" refers to a watersoluble chemical assembly of a metal cation with an anion, a solvate thereof or a co-crystal thereof, in such a way that the total number of positive charges of the metal is equal to the total number of negative charges of the anion. In the context of the present invention, metal salts exhibiting a water solubility superior to 0.05 mole per litre of water are considered suitable. Suitable anions for metal salts are known in the state of the art and include halide anions (for example, fluoride, chloride, bromide and iodide) and oxoanions (such as, for instance, carbonate, hydrogencarbonate, nitrate, sulphate, formate, acetate, acetylacetonate and phosphate). In the context of the present invention, metal salts comprise cations that are known in the state of the art as forming part of methanol synthesis catalysts. This includes, for instance, copper, zinc, aluminium, magnesium, chromium, germanium, silicon, titanium, vanadium and cerium.

In the context of the present invention, the terms "crystalline catalyst" or "crystalline catalyst precursor" and their plurals refer to a catalyst or a catalyst precursor characterized in that the atoms forming the catalyst or the catalyst precursor are ordered in the solid state in such a way that repeating units comprising one or more atoms spatially distributed in a specific manner can be identified. The ensemble of said repeating units forms a crystalline phase. One catalyst or catalyst precursor may have several different crystalline phases wherein the atoms are arranged in a distinct manner from one phase to the other. When the atoms are not spatially ordered in a phase of the catalyst or the catalyst precursor at the solid state, said phase is an amorphous phase. In the context of the present invention, a catalyst or a catalyst precursor comprising in its solid state both amorphous and crystalline phases is said to be "partially crystalline".

When a substance crystallizes from a solution to form a crystalline solid comprising repeating units of atoms spatially order in a specific manner, said repeating units may further comprise solvent molecules or other solutes from the solution.

In the context of the present invention, the term "solvate", when referring to a substance, refers to the fact that at least one crystalline phase of the substance comprises one or more solvent molecules in each repeating unit.

In the context of the present invention, the term "co-crystal", when referring to a substance, refers to the fact that at least one crystalline phase of the substance comprises one or more solute molecules in each repeating unit and, optionally further comprises one or more solvent molecules in each repeating unit.

In the context of the present invention, the term "methanol synthesis catalyst" refers to a catalyst able to produce methanol from a mixture of hydrogen with carbon monoxide and/or carbon dioxide.

In the context of the present invention, the term "low temperature water gas shift catalyst" refers to a catalyst able to promote the water gas shift reaction.

In the context of the present invention, a process carried out under "continuous flow" conditions is characterized in that reactants and reagents are constantly being fed into the reactor and the product is constantly withdrawn from the reactor, such that the residence time of the flowing solution within the reactor corresponds to the reaction time.

As already noted above, according to a first aspect the present invention refers to a method for the preparation of a catalyst precursor comprising the steps of:
(i) heating an aqueous solution of urea with a mixture of metal salts comprising at least a compound of formula Cu(OCOCH₃)₂, a solvate thereof or a co-crystal thereof, to obtain a solid, and wherein the molar amount of urea is at least three times the total molar amount of metal salts;
(ii) separating the solid obtained in step (i) from the aqueous solution;
(iii) calcinating the solid obtained in step (ii).

In a particular embodiment, the method of the present invention allows preparing a crystalline or partially crystalline catalyst precursor.

In a preferred embodiment, as noted above, the method of the present invention is a method for the preparation of a methanol synthesis catalyst precursor.

In the most preferred embodiment, the method of the present invention is a method for the preparation of a crystalline or partially crystalline methanol synthesis catalyst precursor. More particularly, the method of the present invention allows preparing a crystalline or partially crystalline catalyst precursor having an aurichalcite phase.

Preferably, step (i) of the method of the present invention is an homogeneous precipitation step.

In the method of the present invention, preferably, in step (i) the total concentration of metal salts is between 0.1 mole per liter and 0.5 mole per liter, more preferably, in step (i) the total concentration of metal salts is of about 0.25 mole per liter, even more preferably, the total concentration of metal salts is of 0.25 mole per liter.

Also preferably, step (i) of the method of the present invention is carried out in deionized water.

It is contemplated that one or more steps of the method of the present invention is carried out in batch conditions or under continuous flow conditions.

In a preferred embodiment, the mixture of metal salts of step (i) comprises at least a compound of formula Cu(OCOCH₃)₂, a solvate thereof or a co-crystal thereof and salts of metal cations with anions other than nitrate. This embodiment is advantageous as it allows reducing the formation of nitrate contaminating waste during the calcination step or the optional washing step described below.

Suitable anions of metal salts to carry out step (i) of the method of the present invention are oxoanions deriving from sulphur (preferably, sulphate and/or sulphite), phosphorus (preferably, phosphate and/or hydrogenophosphate), or carbon (preferably, formate, acetate, propionate, butyrate, benzoate, oxalate, carbonate and/or hydrogencarbonate). Preferably, the mixture of metal salts of step (i) comprises at least a compound of formula Cu(OCOCH₃)₂, a solvate thereof or a co-crystal thereof and salts of metal cations with anions selected from the group consisting of formate, acetate, propionate, butyrate, benzoate, oxalate, carbonate, and hydrogencarbonate. More preferably, the mixture of metal salts of step (i) comprises at least a compound of formula Cu(OCOCH₃)₂, a solvate thereof or a co-crystal thereof and salts of metal cations with anions selected from the group consisting of formate, acetate, oxalate, carbonate, and hydrogencarbonate. Even more preferably, the mixture of metal salts of step (i) comprises at least a compound of formula Cu(OCOCH₃)₂, a solvate thereof or a co-crystal thereof and salts of metal cations with acetate anions.

In a preferred embodiment, the mixture of metal salts of step (i) comprises at least a compound of formula Cu(OCOCH₃)₂, a solvate thereof or a co-crystal thereof and salts of metal cations wherein the metal is selected from the group consisting of zinc, aluminum, silicon, titanium, vanadium, chromium, manganese and cerium; preferably, the mixture of metal salts of step (i) comprises at least a compound of formula Cu(OCOCH₃)₂, a solvate thereof or a co-crystal thereof and salts of metal cations of one to four metals wherein the metal is selected from the group consisting of zinc, aluminum, silicon, titanium, vanadium, chromium, manganese and cerium; more preferably, the mixture of metal salts of step (i) comprises at least a compound of formula Cu(OCOCH₃)₂, a solvate thereof or a co-crystal thereof and acetate salts of metal cations of one to two metals selected from the group consisting of zinc, aluminum, silicon, titanium, vanadium, chromium, manganese, cerium solvates thereof and co-crystals thereof.

Therefore, preferably, the mixture of metal salts of step (i) comprises at least a compound of formula Cu(OCOCH₃)₂, a solvate thereof or a co-crystal thereof and one to four salts of metal cations with anions other than nitrate, wherein the metal is selected from the group consisting of zinc, aluminum, silicon, titanium, vanadium, chromium, manganese and cerium.

More preferably, the mixture of metal salts of step (i) comprises at least a compound of formula Cu(OCOCH₃)₂, a solvate thereof or a co-crystal thereof and one to two salts of metal cations with anions other than nitrate, wherein the metal is selected from the group consisting of zinc, aluminum, silicon, titanium, vanadium, chromium, manganese and cerium.

Even more preferably, the mixture of metal salts of step (i) comprises at least a compound of formula Cu(OCOCH₃)₂, a solvate thereof or a co-crystal thereof and salts of metal cations with anions other than nitrate, wherein the metal is selected from the group consisting of zinc and aluminum.

More preferably, the mixture of metal salts of step (i) comprises at least a compound of formula Cu(OCOCH₃)₂, a solvate thereof or a co-crystal thereof and salts of metal cations with anions selected from the group consisting of formate, acetate, propionate, butyrate, benzoate, oxalate, carbonate, and hydrogencarbonate and wherein the metal is selected from the group consisting of zinc, aluminum, silicon, titanium, vanadium, chromium, manganese and cerium.

More preferably, the mixture of metal salts of step (i) comprises at least a compound of formula Cu(OCOCH₃)₂, a solvate thereof or a co-crystal thereof and salts of metal cations with anions selected from the group consisting of formate, acetate, oxalate, carbonate, and hydrogencarbonate, and wherein the metal is selected from the group consisting of zinc, aluminum, silicon, titanium, vanadium, chromium, manganese and cerium.

Even more preferably, the mixture of metal salts of step (i) comprises at least a compound of formula Cu(OCOCH₃)₂, a solvate thereof or a co-crystal thereof and acetate salts of metal cations of one to four (even more preferably, one to two) metals selected from the group consisting of zinc, aluminum, silicon, titanium, vanadium, chromium, manganese, cerium, solvates thereof and co-crystal thereof.

The mixture of metal salts in step (i) as explained above, preferably, comprises a compound of formula Zn(OCOCH₃)₂, a solvate thereof or a co-crystal thereof.

Therefore, in a preferred embodiment, the mixture of metal salts comprises substantially Cu(OCOCH₃)₂, a solvate thereof or a co-crystal thereof and Zn(OCOCH₃)₂, a solvate thereof or a co-crystal thereof; more preferably, the mixture of metal salts consists in a mixture of Cu(OCOCH₃)₂, a solvate thereof or a co-crystal thereof and Zn(OCOCH₃)₂, a solvate thereof or a co-crystal thereof. The mixture of metal salts may in particular further comprise a salt of aluminium with an anion other than nitrate, such as aluminium monoacetate, aluminium diacetate and aluminium triacetate.

In addition, it is contemplated that the molar fraction of the compound of formula Cu(OCOCH₃)₂, a solvate thereof or a co-crystal thereof in the mixture of metal salts of step (i) is at least 20% (in mole/mole, hereinafter M/M); preferably it is comprised in the range of 25% (M/M) to 75% (M/M); preferably it is comprised in the range of 30% (M/M) to 70% (M/M); more preferably it is about 50% (M/M); and even more preferably it is 50% (M/M).

Therefore, preferably, the mixture of metal salts in step (i) comprises a compound of formula Zn(OCOCH₃)₂, a solvate thereof or a co-crystal thereof and the molar fraction of the compound of formula Cu(OCOCH₃)₂, a solvate thereof or a co-crystal thereof in the mixture of metal salts of step (i) is at least 20% (M/M); preferably the molar fraction of the compound of formula Cu(OCOCH₃)₂, a solvate thereof or a co-crystal thereof in the mixture of metal salts of step (i) is comprised in the range of 25% (M/M) to 75% (M/M); preferably the molar fraction of the compound of formula Cu(OCOCH₃)₂, a solvate thereof or a co-crystal thereof in the mixture of metal salts of step (i) is comprised in the range of 30% (M/M) to 70% (M/M); more preferably, the molar fraction of the compound of formula Cu(OCOCH₃)₂, a solvate thereof or a co-crystal thereof in the mixture of metal salts of step (i) is about 50% (M/M); and even more preferably, the molar fraction of the compound of formula Cu(OCOCH₃)₂, a solvate thereof or a co-crystal thereof in the mixture of metal salts of step (i) is 50% (M/M).

Hence, in a most preferred embodiment, the mixture of metal salts in step (i) consists of a mixture of Cu(OCOCH₃)₂ and Zn(OCOCH₃)₂, and the molar fraction of the compound of formula Cu(OCOCH₃)₂, a solvate thereof or a co-crystal thereof in the mixture of metal salts of step (i) is at least 20% (M/M); preferably the molar fraction of the compound of formula Cu(OCOCH₃)₂, a solvate thereof or a co-crystal thereof in the mixture of metal salts of step (i) is comprised in the range of 25% (M/M) to 75% (M/M); preferably the molar fraction of the compound of formula Cu(OCOCH₃)₂, a solvate thereof or a co-crystal thereof in the mixture of metal salts of step (i) is comprised in the range of 30% (M/M) to 70% (M/M); more preferably, the molar fraction of the compound of formula Cu(OCOCH₃)₂, a solvate thereof or a co-crystal thereof in the mixture of metal salts of step (i) is about 50% (M/M); and even more preferably, the molar fraction of the compound of formula Cu(OCOCH₃)₂, a solvate thereof or a co-crystal thereof in the mixture of metal salts of step (i) is 50% (M/M).

In a further preferred embodiments, the mixture of metal salts comprises Cu(OCOCH₃)₂ and Zn(OCOCH₃)₂, solvates thereof or co-crystals thereof, and the molar ratio of Cu(OCOCH₃)₂ to Zn(OCOCH₃)₂ in the mixture of metal salts is between 3:1 and 1:3; preferably the molar ratio of Cu(OCOCH₃)₂ to Zn(OCOCH₃)₂ is between 2:1 and 1:2; and more preferably the molar ratio of Cu(OCOCH₃)₂ to Zn(OCOCH₃)₂ is 1:1. More preferably, the mixture of metal salts consists of Cu(OCOCH₃)₂ and Zn(OCOCH₃)₂, solvates thereof or co-crystals thereof, and the molar ratio of Cu(OCOCH₃)₂ to Zn(OCOCH₃)₂ in the mixture of metal salts is between 3:1 and 1:3; preferably the molar ratio of Cu(OCOCH₃)₂ to Zn(OCOCH₃)₂ is between 2:1 and 1:2; and more preferably the molar ratio of Cu(OCOCH₃)₂ to Zn(OCOCH₃)₂ is 1:1.

In a particular embodiment, the molar amount of urea used in step (i) of the method of the present invention is between 3 and 1000 times the total molar amount of the mixture of metal salts. Preferably, the molar amount of urea used in step (i) of the method of the present invention is between 3 and 100 times the total molar amount of the mixture of metal salts. More preferably, the molar amount of urea used in step (i) of the method of the present invention is between 3 and 50 times the total molar amount of the mixture of metal salts. More preferably, the molar amount of urea used in step (i) of the method of the present invention is between 3 and 20 times the total molar amount of the mixture of metal salts. More preferably, the molar amount of urea used in step (i) of the method of the present invention is between 3 and 10 times the total molar amount of the mixture of metal salts. Even more preferably, the molar amount of urea used in step (i) of the method of the present invention 10 times the total molar amount of the mixture of metal salts.

In another particular embodiment, the aqueous solution of step (i) has a pH of 5 or higher. Preferably, the aqueous solution of step (i) has a pH between 5 and 7.

In another particular embodiment of the first aspect of the invention, the total concentration of metal salts in step (i) is between 0.1 and 0.5 M; more preferably, it is of 0.25 M.

In another particular embodiment, step (i) of the method of the present invention is carried out at temperature between 70 °C and 90 °C, preferably of about 80 °C, even more preferably of 80 °C.

In another particular embodiment, step (i) of the method of the present invention is carried out during a time of at least 12 hours. Preferably, step (i) of the method of the present invention is carried out during a time of between 12 and 24 hours. Said duration of step (i) is advantageous as it comprises an aging period allowing for the formation of specific crystalline phases within the formed precipitate, which will give rise to crystallite structures upon calcination of the isolated solid. Said aging phase of step (i) is carried out at the same temperature as step (i) and as described above.

In another particular embodiment, step (ii) of the method of the present invention is carried out by a separation method selected from any solid-liquid separation technique known in the state of the art, preferably, step (ii) of the method of the present invention is carried out by a separation method selected from the group consisting of decantation, centrifugation and filtration. More preferably, step (ii) of the method of the present invention is carried out by filtration.

According to the methods described in the state of the art, and in order to maintain catalyst efficiency, known methods for the preparation of catalyst precursors further comprise as essential step the washing of the solid obtained in step (ii) with water prior to performing step (iii).

In the present invention, such washing step is optional but not essential as it is directly derivable from the results included below in the examples.

Hence, it is contemplated that the method of the present invention further comprises a step (ii') of washing the solid obtained in step (ii) with water prior to step (iii).

However, in the most preferred embodiment the method of the present invention omits such optional washing step. Inventors have found that, surprisingly, the catalytic activity of catalyst prepared in accordance with the method of the present invention wherein such washing step is omitted, is not affected and, hence, the omittance of said washing step is beneficial or not prejudicial to the efficiency of the catalyst.

As already noted above, in a particular embodiment the method of the present invention further comprises step (ii') as defined above, wherein the solid obtained in step (ii) is washed with water, preferably with deionized water, prior to performing step (iii). This is advantageous as it allows removing ammonium salts and non-metallic salts from the precipitate and is known to promote the formation of small crystallite in the catalyst precursor. When nitrate, halide, or sulphate salts of metals are used in step (i), it is preferred that the step (ii') of washing is carried out. This washing step is beneficial to the catalytic activity of the catalyst obtained out of the produced catalyst precursor. When such washing step is carried out in the method of the invention providing a catalyst with comparable efficiency as a catalyst prepared form copper (II) nitrate, per each mole of copper(II) acetate engaged in step (i), about two moles of nitrates in wastewater are avoided, which is advantageous as it generates a less contaminating wastewater.

When acetate salts are used in step (i) of the method of the present invention, step (ii') is optional, as the catalytic activity of the formed compound remains unchanged whether the solid obtained in step (ii) is washed or not. When acetate salts, are used in the step (i) of the method of the present invention, step (ii') is preferably omitted.

In some particular embodiments, step (iii) is carried out by heating the solid at a temperature between 250 °C and 350 °C; preferably, step (iii) is carried out by heating the solid at a temperature of 300 °C.

In some particular embodiments, step (iii) is carried out by heating the solid during a period of at least thirty minutes; preferably, step (iii) is carried out by heating the solid during a period of one hour, in particular at a temperature of 300 °C.

In some particular embodiments, step (iii) is carried out using a heating rate of between 1 and 3 °C per minute; preferably using a heating rate of 2 °C per minute.

Consequently, in a most preferred embodiment, the method of the present invention does not comprise step (ii') (it does comprise steps (i), (ii) and (iii)). More preferably, the method of the present invention consists of steps (i), (ii) and (iii) as defined above and below. As mentioned above, this is particularly the case when the mixture of metal salts consists of metal salts with acetate salts of metal cations.

In some particular embodiments, the method of the present invention consists of steps (i), (ii) and (iii), wherein the mixture of metals salts of step (i) comprises a compound of formula Cu(OCOCH₃)₂, a solvate thereof or a co-crystal thereof and salts of metal cations with anions selected from the group consisting of formate, acetate, oxalate, carbonate, and hydrogencarbonate.

In some particular embodiments, the method of the present invention consists of the steps (i), (ii) and (iii), wherein the mixture of metals salts of step (i) comprises a compound of formula Cu(OCOCH₃)₂, a solvate thereof or a co-crystal thereof and salts of metal cations with anions selected from the group consisting of formate, acetate, oxalate, carbonate, and hydrogencarbonate and wherein the metal is selected from the group consisting of zinc, aluminum, silicon, titanium, vanadium, chromium, manganese, cerium, solvates thereof and co-crystal thereof.

In some particular embodiments, the method of the present invention consists of the steps (i), (ii) and (iii), wherein the mixture of metal salts of step (i) comprises at least a compound of formula Cu(OCOCH₃)₂, a solvate thereof or a co-crystal thereof and acetate salts of one to two metals selected from the group consisting of zinc, aluminum, silicon, titanium, vanadium, chromium, manganese, cerium, solvates thereof and co-crystal thereof.

In a preferred embodiment, the method of the present invention consists of the steps (i), (ii) and (iii), wherein the mixture of metal salts used in step (i) comprises substantially Cu(OCOCH₃)₂ and Zn(OCOCH₃)₂. In a preferred embodiment, the method of the present invention consists of the steps (i), (ii) and (iii), wherein the mixture of metal salts used in step (i) consists of a mixture of Cu(OCOCH₃)₂ and Zn(OCOCH₃)₂.

In some particular preferred embodiments, the present invention refers to a method for the preparation of a crystalline or partially crystalline methanol synthesis catalyst precursor comprising the steps of:
(i) heating an aqueous solution of urea with a mixture of metal salts comprising at least a compound of formula Cu(OCOCH₃)₂, a solvate thereof or a co-crystal thereof, to obtain a solid, and wherein:
   the temperature of the aqueous solution is 80 °C;
   the mixture of metal salts comprises substantially Cu(OCOCH₃)₂ and
   Zn(OCOCH₃)₂, solvates thereof or co-crystals thereof, and the molar ratio of Cu(OCOCH₃)₂ to Zn(OCOCH₃)₂ in the mixture of metal salts is comprised from 3:1 to 1:3;
   the molar amount of urea is between three and ten times the total molar amount of the mixture of metal salts;
   the reaction time is at least 12 hours;
(ii) separating the solid obtained in step (i) from the aqueous solution by filtration; and
(iii) calcinating the solid obtained in step (ii) at a temperature of between 250 °C and 350 °C.

In some particular preferred embodiments, the present invention refers to a method for the preparation of a crystalline or partially crystalline methanol synthesis catalyst precursor comprising the steps of:
(i) heating an aqueous solution of urea with a mixture of metal salts comprising at least a compound of formula Cu(OCOCH₃)₂, a solvate thereof or a co-crystal thereof, to obtain a solid, wherein:
   the temperature of the aqueous solution is 80 °C;
   the mixture of metal salts consists of Cu(OCOCH₃)₂ and Zn(OCOCH₃)₂, solvates thereof or co-crystals thereof, and the molar ratio of Cu(OCOCH₃)₂ to Zn(OCOCH₃)₂ in the mixture of metal salts is between 3:1 and 1:3;
   the molar amount of urea is between three and ten times the total molar amount of the mixture of metal salts;
   the reaction time is at least 12 hours;
(ii) separating the solid obtained in step (i) from the aqueous solution by filtration; and
(iii) calcinating the solid obtained in step (ii) at a temperature of between 250 °C and 350 °C.

In some particular preferred embodiments, the present invention refers to a method for the preparation of a crystalline or partially crystalline methanol synthesis catalyst precursor comprising the steps of:
(i) heating an aqueous solution of urea with a mixture of metal salts comprising at least a compound of formula Cu(OCOCH₃)₂, a solvate thereof or a co-crystal thereof, to obtain a solid, wherein:
   the temperature of the aqueous solution is 80 °C;
   the mixture of metal salts consists of Cu(OCOCH₃)₂ and Zn(OCOCH₃)₂,
   solvates thereof or co-crystals thereof, and the molar ratio of Cu(OCOCH₃)₂ to Zn(OCOCH₃)₂ in the mixture of metal salts is 1:1;
   the molar amount of urea is between three and ten times the total molar amount of the mixture of metal salts;
   the reaction time is at least 12 hours;
(ii) separating the solid obtained in step (i) from the aqueous solution by filtration; and
(iii) calcinating the solid obtained in step (ii) at a temperature between 250 °C and 350 °C.

In some further particular preferred embodiments, the present invention refers to a method for the preparation of a crystalline or partially crystalline methanol synthesis catalyst precursor consisting in the steps of:
(i) heating an aqueous solution of urea with a mixture of metal salts comprising at least a compound of formula Cu(OCOCH₃)₂, a solvate thereof or a co-crystal thereof, to obtain a solid, wherein:
   the temperature of the aqueous solution is 80 °C;
   the mixture of metal salts comprises substantially Cu(OCOCH₃)₂ and Zn(OCOCH₃)₂, solvates thereof or co-crystals thereof, and the molar ratio of Cu(OCOCH₃)₂ to Zn(OCOCH₃)₂ in the mixture of metal salts is comprised from 3:1 to 1:3;
   the molar amount of urea is between three and ten times the total molar amount of the mixture of metal salts;
   the reaction time is at least 12 hours;
(ii) separating the solid obtained in step (i) from the aqueous solution by filtration; and
(iii) calcinating the solid obtained in step (ii) at a temperature of between 250 °C and 350 °C.

In some further particular preferred embodiments, the present invention refers to a method for the preparation of a crystalline or partially crystalline methanol synthesis catalyst precursor consisting in the steps of:
(i) heating an aqueous solution of urea with a mixture of metal salts comprising at least a compound of formula Cu(OCOCH₃)₂, a solvate thereof or a co-crystal thereof, to obtain a solid, wherein:
   the temperature of the aqueous solution is 80 °C;
   the mixture of metal salts consists of Cu(OCOCH₃)₂ and Zn(OCOCH₃)₂,
   solvates thereof or co-crystals thereof, and the molar ratio of Cu(OCOCH₃)₂ to Zn(OCOCH₃)₂ in the mixture of metal salts is between 3:1 and 1:3;
   the molar amount of urea is between three and ten times the total molar amount of the mixture of metal salts;
   the reaction time is at least 12 hours;
(ii) separating the solid obtained in step (i) from the aqueous solution by filtration; and
(iii) calcinating the solid obtained in step (ii) at a temperature of between 250 °C and 350 °C.

In some further particular preferred embodiments, the present invention refers to a method for the preparation of a crystalline or partially crystalline methanol synthesis catalyst precursor consisting in the steps of:
(i) heating an aqueous solution of urea with a mixture of metal salts comprising at least a compound of formula Cu(OCOCH₃)₂, a solvate thereof or a co-crystal thereof, to obtain a solid, wherein:
   the temperature of the aqueous solution is 80 °C;
   the mixture of metal salts consists of Cu(OCOCH₃)₂ and Zn(OCOCH₃)₂, solvates thereof or co-crystals thereof, and the molar ratio of Cu(OCOCH₃)₂ to Zn(OCOCH₃)₂ in the mixture of metal salts is 1:1;
   the molar amount of urea is between three and ten times the total molar amount of the mixture of metal salts;
   the reaction time is at least 12 hours;
(ii) separating the solid obtained in step (i) from the aqueous solution by filtration; and
(iii) calcinating the solid obtained in step (ii) at a temperature of between 250 °C and 350 °C; preferably of 300 °C.

In addition, it is contemplated that the method of the present invention further comprises one, two or three of the following steps of:
(iv) pelletizing the solid obtained in step (iii);
(v) crushing the solid obtained in step (iii) or in step (iv); and
(vi) in the solid obtained in step (iii) or in step (iv) or in step (v), selecting the solid particles having a diameter lower than 1000 µm; preferably the particles having a diameter lower than 500 µm; and more preferably the particles having a diameter of between 100 µm and 300 µm.

As described above, the method of the invention may further comprise step (ii') as defined above, wherein the solid obtained in step (ii) is washed with water, preferably with deionized water, prior to performing step (iii). When salts of metals with an anion selected from the group consisting of nitrate, halide and oxoanions deriving from sulphur are used in step (i), step (ii') of washing is recommended. When metal salts with certain anions, such as nitrate, chloride or sulphate are used in step (i), it is known in the state of the art that the resulting catalyst exhibits lower activity if the catalyst precursor is not washed prior to the calcination step. In such cases, the efficiency of the catalyst improves provided that the washing step (ii') is carried out. The method of the invention is advantageous since, when it comprises a washing step (ii'), it generates a wastewater with a reduced amount of nitrate if compared with the methods known in the art, while providing an efficient catalyst. When acetate salts are used in the step (i) of the method of the present invention, step (ii') is preferably omitted. It is advantageous since the omission of the washing step makes the method of preparation of the catalyst precursor cleaner and shorter, while providing a catalyst composition whose catalytic activity is not affected by the omission of the washing step.

As noted previously, in the state of the art, when salts other than copper (II) nitrate salts are used in the preparation of a copper-based methanol synthesis catalyst precursor, the efficiency of the resulting catalyst is typically lower than for catalysts prepared from copper (II) nitrate salts. As described herein, the method of the present invention provides an improved preparation method for the preparation of a copper-based catalyst precursor which, without using salts of copper (II) nitrate, provides a catalyst precursor that when activated provides a catalyst which surprisingly exhibits an efficiency that is similar or comparable to the efficiency of catalysts prepared using copper (II) nitrate as metal precursor, while presenting the further advantage of generating a less contaminating wastewater, or no wastewater at all. The invention thus provides an improved method for the preparation of a copper-based catalyst precursor wherein the method presents both a reduced need for waste management and a reduced environmental impact, said method allowing preserving the level of activity and efficiency of the catalyst. Therefore, the present invention solves the problems present in the state of the art and mentioned above.

Thus, a second aspect the present invention refers to a catalyst precursor obtained by means of a method of the present invention, this is, a method for the preparation of a catalyst precursor as described above.

As noted above, the catalyst precursor of the present invention is a methanol synthesis catalyst precursor or a catalyst precursor for the low temperature water gas shift reaction. Preferably, it is a methanol synthesis catalyst precursor.

Also, preferably, the catalyst precursor of the present invention is a crystalline or partially crystalline catalyst precursor.

Therefore, in a most preferred embodiment, the catalyst precursor of the present invention is a crystalline or partially crystalline methanol synthesis catalyst precursor. Even more preferably, the catalyst precursor of the present invention is a crystalline or partially crystalline catalyst precursor comprising an aurichalcite crystalline phase.

It is also contemplated in some embodiments of the invention that the catalyst precursor of the present invention is characterized by having a powder X-ray diffraction pattern comprising one or more peaks at 2θ with values selected from 27.5, 30.9, 33.0, 34.2, 37.4, 41.9 and 46.3 degrees, preferably all peaks. In other embodiments of the invention, the catalyst precursor is characterized by having a powder X-ray diffraction spectrum as described in Figure 1(a) or in Figure 1(b).

It is also contemplated in some embodiments of the invention that the catalyst precursor of the present invention is characterized by having a crystallite size of between 8 and 9 nm.

All the particular and preferred embodiments described for the method of the invention provide specific catalyst precursors that individually represent particular embodiments of the second aspect of the invention.

As already explained above, the catalyst precursors of the present invention is obtained or prepared without using copper (II) nitrate as metal precursor, but still (and contrary to what is stated in the state of the art), provides for catalysts which exhibit an efficiency that is similar or comparable to the efficiency of catalysts prepared using copper (II) nitrate as metal precursor, while presenting the further advantage of generating a less contaminating wastewater, or no wastewater at all. The invention thus provides an improved catalyst precursor wherein in the preparation of said catalyst precursor there is a reduced need for waste management and a reduced environmental impact and the level of activity and efficiency of the catalyst obtained is maintained. Therefore, the present invention solves the problems present in the state of the art and mentioned above.

In a third aspect, the present invention refers to a method for the preparation of a catalyst based on the activation of a catalyst precursor obtained in accordance with the method for the preparation of a catalyst precursor of the present invention as described above.

As known in the art, the method for the preparation of a catalyst precursor of the present invention provides a catalyst precursor that requires to be activated in order to provide the actual active catalyst for the hydrogenation processes. This activation process typically reduces the CuO crystalline phase in the catalyst precursor to Cu active sites, which are prompt to adsorb hydrogen and carbon dioxide and transform the molecules. The activation step typically consists in submitting the catalyst precursor obtainable by the method for the preparation of a catalyst precursor of the present invention to a hydrogen atmosphere, preferably in the absence of oxygen.

Therefore, in a preferred embodiment, the method for the preparation of a catalyst of the present invention comprises the steps described above for the method for the preparation of a catalyst precursor and, afterwards, a final step of activation of the catalyst precursor. Preferably, as noted above, said step of activation of the catalyst precursor consists in submitting the catalyst precursor to a hydrogen atmosphere, preferably in the absence of oxygen.

In preferred embodiments of the third aspect of the invention, said step of activation of the catalyst precursor consists in submitting the catalyst precursor to a hydrogen atmosphere at a temperature between 200 °C and 400 °C; preferably at a temperature of 260 °C. Preferably, said step of activation is carried out in the absence of oxygen.

In preferred embodiments, said step of activation has a duration of at least one hour, preferably, it has a duration of two hours.

As noted previously, in the state of the art, when salts other than copper (II) nitrate salts are used in the preparation of a copper-based methanol synthesis catalyst precursor, the efficiency of the resulting catalyst is typically lower than for catalysts prepared from copper (II) nitrate salts. However, the method of the present invention provides an improved preparation method for the preparation of a copper-based catalyst which, without using salts of copper (II) nitrate, provides a copper-based catalyst which surprisingly exhibits an efficiency that is similar or comparable to the efficiency of catalysts prepared using copper (II) nitrate as metal precursor, while presenting the further advantage of generating a less contaminating wastewater, or no wastewater at all. The invention thus provides an improved method for the preparation of a copper-based catalyst wherein the method presents both a reduced need for waste management and a reduced environmental impact, said method allowing preserving the level of activity and efficiency of the catalyst. Therefore, the present invention solves the problems present in the state of the art and mentioned above.

In a fourth aspect, the present invention refers to a catalyst obtained by means of a method for the preparation of a catalyst of the present invention (this is, as described above).

The catalyst of the present invention is, preferably, a methanol synthesis catalyst.

Also, preferably, the catalyst of the present invention is a crystalline or partially crystalline catalyst.

Therefore, in a most preferred embodiment, the catalyst of the present invention is a crystalline or partially crystalline methanol synthesis catalyst.

In another embodiment, the catalyst of the present invention is a crystalline or partially crystalline catalyst for the low temperature water gas shift reaction.

As already explained above, the catalyst of the present invention is obtained or prepared without using copper (II) nitrate as metal precursor, but still (and contrary to what is stated in the state of the art), exhibit an efficiency that is similar or comparable to the efficiency of catalysts prepared using copper (II) nitrate as metal precursor, while presenting the further advantage of generating a less contaminating wastewater, or no wastewater at all. The invention thus provides an improved catalyst wherein in the preparation of said catalyst there is a reduced need for waste management and a reduced environmental impact and the level of activity and efficiency of the catalyst obtained is maintained. Therefore, the present invention solves the problems present in the state of the art and mentioned above.

In a fifth aspect, the present invention refers to a composition comprising a catalyst or a catalyst precursor of the present invention (this is, a composition comprising a catalyst or a catalyst precursor obtained by means of a method of the present invention, in accordance with what is explained above).

Hereinafter, the composition comprising a catalyst of the present invention is referred as catalyst composition.

Also hereinafter, the composition comprising a catalyst precursor of the present invention is referred as catalyst precursor composition.

The catalyst and the catalyst precursor, both of the present invention, are as explained above.

As described in the Examples section, the catalyst of the present invention is useful as a methanol synthesis catalyst, in particular via high-pressure hydrogenation of carbon dioxide.

The compositions of the present invention show the technical effects explained above for the catalyst precursor and the catalyst, both of the present invention.

A sixth aspect of the present invention, therefore, relates to the use of a catalyst or a catalyst composition, both of the present invention, as catalyst in a method for the preparation of methanol by hydrogenation of carbon dioxide or for the low temperature water gas sift reaction. A preferred embodiment of the sixth aspect of the present invention relates to the use of a catalyst or a catalyst composition of the present invention as catalyst in a method for the preparation of methanol by hydrogenation of carbon dioxide.

In a seventh aspect, the present invention, therefore, relates to a method for the preparation of methanol by hydrogenation of carbon dioxide comprising the step of contacting carbon dioxide with hydrogen in the presence of a catalytically effective amount of a catalyst or a catalyst, both of the present invention.

Suitable conditions are known in the art to carry out such a method, such as those described in Bansode et al. Journal of Catalysis 343 (2016) 127-132, or in the PCT Patent applications WO2017140800 and WO2013171239.

Thus, in particular embodiments of the seventh aspect of the present invention, the method for the preparation of methanol by hydrogenation of carbon dioxide comprises contacting carbon dioxide with hydrogen in the presence of a catalytically effective amount of the catalyst or the catalyst composition, both of the present invention, wherein the pressure is equal to or higher than 150 bar; the temperature is between 200 and 330 °C; and the space velocity is at least 500 h⁻¹. The term "space velocity is also referred to herein as "gas hourly space velocity".

In other particular embodiments, the method for the preparation of methanol by hydrogenation of carbon dioxide comprises contacting carbon dioxide with hydrogen in the presence of a catalytically effective amount of the catalyst or the catalyst composition of the present invention wherein the molar ratio of hydrogen to carbon dioxide is between about 3:1 and about 10:1; preferably, it is of about 3:1; even more preferably, it is of 3:1.

In a more particular embodiment, the method for the preparation of methanol by hydrogenation of carbon dioxide comprises contacting carbon dioxide with hydrogen in the presence of a catalytically effective amount of the catalyst or the catalyst composition, both of the present invention, wherein the method is carried out at a pressure of at least 200 bar, preferably at a pressure between 300 and 400 bar; more preferably at a pressure of about 330 bar; and even more preferably, at a pressure of 330 bar, being said pressure the sum of partial pressures of carbon dioxide and hydrogen. In such conditions, the method for the preparation of methanol by hydrogenation of carbon dioxide of the present invention allows preparing methanol in high conversion of carbon dioxide and selectivity of formation of methanol.

In a more particular embodiment, the method for the preparation of methanol by hydrogenation of carbon dioxide comprises contacting carbon dioxide with hydrogen in the presence of a catalytically effective amount of the catalyst or of the catalyst composition, both of the present invention, wherein the method is carried out at a temperature of about 280 °C, more preferably at a temperature of 280 °C.

In more particular embodiments, the method for the preparation of methanol by hydrogenation of carbon dioxide comprises contacting carbon dioxide with hydrogen in the presence of a catalytically effective amount of the catalyst or of the catalyst composition, both of the present invention, wherein the space velocity is from 5000 h⁻¹ to 100000 h⁻¹, preferably the space velocity is from 5000 h⁻¹ to 20000 h⁻¹, more preferably it is of about 8500 h⁻¹, even more preferably, it is of 8500 h⁻¹. In such conditions, the method for the preparation of methanol by hydrogenation of carbon dioxide of the present invention allows preparing methanol in high weight-time yields.

In more particular embodiments of the present invention, the method for the preparation of methanol by hydrogenation of carbon dioxide comprises contacting carbon dioxide with hydrogen in the presence of a catalytically effective amount of the catalyst or a catalyst composition, both of the present invention, wherein:
the molar ratio of hydrogen to carbon dioxide is between about 3:1 and about 10:1; preferably, it is of about 3:1; more preferably, it is 3:1;
the method is carried out at a pressure of at least 200 bar, preferably at a pressure between 300 and 400 bar; more preferably at a pressure of about 330 bar; even more preferably at a pressure of 330 bar, being said pressure the sum of partial pressures of carbon dioxide and hydrogen;
the method is carried out at a temperature of about 280 °C, more preferably at a temperature of 280 °C; and
the space velocity is between 5000 h⁻¹ and 20000h⁻¹, preferably it is of about 8500 h⁻¹, more preferably is of 8500 h⁻¹.

Such conditions are advantageous as they allow converting efficiently carbon dioxide into methanol in one sole-pass through the reactor, which allows for the production of high amounts of methanol in a reduced period of time and using a reduced reactor space thanks to high-pressure operation.

Throughout the description and claims the word "comprises" and variations of the word, are not intended to exclude other technical features, additives, components or steps. Furthermore, the word "comprise" encompasses the case of "consisting of". Additional objects, advantages and features of the present invention will become apparent to those skilled in the art upon examination of the description or may be learned by practice of the present invention. The following examples are provided by way of illustration, and they are not intended to be limiting of the present invention. Furthermore, the present invention covers all possible combinations of particular and preferred embodiments described herein.

### EXAMPLES

### Example 1: Preparation of a Cu/ZnO catalyst precursor by homogeneous precipitation of acetate salts of copper and zinc induced by urea

Procedure 1: An aqueous solution containing Cu(CH₃COO)₂, Zn(CH₃COO)₂, and urea was freshly prepared at room temperature. The total concentration of Cu(CH₃COO)₂ and Zn(CH₃COO)₂ in the solution was kept constant at 0.25 M. The molar ratio between Cu and Zn salts in the solution was 50%/50% (M/M), while the molar ratio of urea to the sum of the molar amounts of Cu and Zn salts was 10:1. The solution was added to a round-bottom flask and heated to 80 °C using a heating mantle and while stirring at 1000 rpm using a magnetic stirrer. After 24 hours of precipitation process, the obtained precipitate was filtered, optionally washed with adequate deionized water, and dried at 80 °C overnight. The dried precursor was calcined at 300 °C for 1 hour by heating at a rate of 2 °C/min. The calcined catalyst powder was pelletized with a pressing die, crushed in a mortar, and sieved to the size of 100-300 µm. The pelletization pressure was 370 kg/cm² or ca. 363 bar, similar to the reaction pressure, to ensure no deformation of the catalyst pellets during gas pressurization.

With the procedure mentioned above (with the appropriate adaptations), the catalyst precursors noted in table 1 were prepared.

**Table 1. Details of the method of preparation for the catalysts precursors prepared in Example 1.**

| Catalyst precursor | Washing step | Molar ratio of Cu(CH₃COO)₂ to Zn(CH₃COO)₂ (%Cu/%Zn) | Molar ratio of urea to metal salts |
|---|---|---|---|
| 1 | Yes | 50/50 | 4 |
| 2 | Yes | 50/50 | 7 |
| 3 | Yes | 50/50 | 10 |
| 3' | No | 50/50 | 10 |
| 4 | Yes | 25/75 | 10 |
| 4' | No | 25/75 | 10 |
| 5 | Yes | 75/25 | 10 |
| 5' | No | 75/25 | 10 |

Catalyst precursors 1, 2, 3, 4, 5 and catalyst precursors 3', 4' and 5' were prepared following Procedure 1. In the preparation of catalyst precursors 1, 2, 3, 4, 5 the optional washing step of the filtered catalyst was carried out, while in the preparation of catalyst precursors 3', 4' and 5', such washing step was omitted.

### Comparative preparative example: Preparation of a Cu/ZnO catalyst precursor by homogeneous precipitation induced by urea of nitrate salts of copper and zinc (comparative catalyst 1 and comparative catalyst 1')

The Cu(NO₃)₂·3H₂O, Zn(NO₃)₂·6H₂O and urea were purchased from Sigma-Aldrich. 100 mL aqueous solution containing Cu(NO₃)₂, Zn(NO₃)₂ and urea was freshly prepared and kept at room temperature. The total concentration of Cu(NO₃)₂ and Zn(NO₃)₂ in the solution was kept constant at 0.25 M. The ratio between Cu and Zn in the solution was 50%/50%, while the molar ratio of urea to Cu and Zn 10:1. The solution was added to a round-bottom flask and heated to 80 °C using a heating mantle and while stirring at 1000 rpm using a magnetic stirrer. After 24 hours of precipitation process, the obtained precipitate was slowly cooled down to room temperature and aged overnight in the mother liquor under vigorously stirring. The aged precipitate was filtered, optionally washed with adequate deionized water, and dried at 80 °C overnight. The dried precursor was calcined at 300 °C for 1 hour by heating at a rate of 2 °C/min. The calcined catalyst powder was pelletized with a pressing die, crushed in a mortar, and sieved to the size of 100-300 µm. The pelletization pressure was 370 kg/cm² or ca. 363 bar, similar to the reaction pressure, to ensure no deformation of the catalyst pellets during gas pressurization.

In the preparation of comparative catalyst 1, the optional washing step of the filtered catalyst was carried out, while in the preparation of comparative catalyst 1', such washing step was omitted.

The prepared catalysts precursors were also compared with the standard commercial methanol synthesis catalyst precursor Cu/ZnO/Al₂O₃ (Alfa Aesar Product No.: 45776) - hereinafter, comparative catalyst 2.

### Characterization of catalyst precursors

Surface area and pore volume were determined by N₂ adsorption at -196 °C using a Quantachrome Autosorb iQ. Both surface and pore volume were obtained from multipoint Brunauer-Emmett-Teller (BET) method. Prior to the analysis, the samples were degassed to eliminate volatile adsorbents on the surface at 250°C for 6 hours. The quantity of gas adsorbed onto or desorbed from a solid surface was measured at 15 equilibrium vapor pressure (P/Pₒ) values from 0.0053 to 0.3014 by the static volumetric method.

The obtained results appear summarized in table 2.

**Table 2. Results obtained for crystallite size in example 1.**

| Catalyst precursors | Crystallite size (nm) | |
|---|---|---|
| | CuO | ZnO |
| Comparative catalyst 1 | 8.1 | 9.6 |
| Comparative catalyst 1' | 18.7 | 44.4 |
| Comparative catalyst 2 | 8.2 | 9.2 |
| 1 | 8.3 | 8.2 |
| 2 | 8.5 | 8.1 |
| 3 | 8.6 | 8.2 |
| 3' | 8.2 | 8.2 |
| 4 | 8.2 | 8.2 |
| 4' | 8.1 | 11.1 |
| 5 | 8.2 | 8.5 |
| 5' | 8.2 | 8.7 |

The results of Table 2 indicate that when acetate salts of copper and zinc are precipitated in the presence of urea, the washing step can be omitted, as the resulting catalyst precursor exhibits small particle size.

### Example 2: Methanol synthesis by high-pressure CO₂ hydrogenation

General procedure: The CO₂ hydrogenation was carried out in a fixed bed reactor (Outer Diameter of 1/4" and Internal Diameter of 0.12" or 3.048 mm) packed with 200 mg of catalyst precursor (bed length of about 20 mm). In order to achieve plug flow condition and to eliminate back filling, channelling and wall effect, the criteria L_{bed}/Dₚₑₗₗₑₜ > 50, ID_{reactor}/D_{Pellet} > 10, and L_{bed}/ ID_{reactor} > 3 were fulfilled, wherein L_{bed} refers to the length of the fixed-bed of catalyst, Dₚₑₗₗₑₜ refers to the average diameter of the pelletized catalyst particle, and ID_{reactor} refers to the internal diameter of the plug-flow reactor. Prior to running the reaction, the catalyst precursor was activated. To this end, the catalyst precursor was reduced in situ using 90% (V/V) H₂/Ar at temperature 260 °C with flow rate 20 mL/min for 2 hours. After reduction, the catalyst was cooled down slowly to room temperature under same atmosphere. The premixed feed of 68.85% H₂, 23.08% CO₂ and 8.07% Ar was introduced to reactor and pressurized to 360 bars (total pressure) using a syringe pump (molar or volume percentages). The gas hourly space velocity (GHSV) was 8500 hr⁻¹. The catalyst pellet of this size is suitable for GHSV up to 20000-30000 hr⁻¹.

Table 3 below shows the conversion of carbon dioxide and the selectivity to methanol depending of the catalyst used in the methanol synthesis process. Table 3 indicates that the catalysts prepared according to the method of the present invention are efficient methanol synthesis catalysts and are potentially superior than the commercial methanol synthesis catalyst or other catalysts prepared according to methods described in the state of the art. It also shows that when the washing step is omitted in the preparation of the catalyst, the efficiency of the prepared catalyst is not dramatically reduced, as observed using preparation methods of the state of the art.

**Table 3. Conversion of carbon dioxide, the selectivity to methanol and methanol yield depending of the catalyst used in the methanol synthesis process of example 2.**

| Catalyst | Temperature (°C) | CO₂ conversion (%) | Selectivity to methanol (%) | Methanol yield (mg of methanol per gram of catalyst per hour) |
|---|---|---|---|---|
| Comparative catalyst 1 | 220 | 22.8 | 70.3 | 264.6 |
| | 240 | 38.2 | 86.3 | 544.6 |
| | 260 | 62.2 | 96.1 | 986.5 |
| | 280 | 71.8 | 97.2 | 1152.1 |
| | 300 | 63.9 | 95.3 | 1004.7 |
| Comparative catalyst 1' | 220 | 1.5 | 69.0 | 17.1 |
| | 240 | 15.6 | 63.9 | 164.5 |
| | 260 | 30.4 | 67.0 | 336.1 |
| | 280 | 38.3 | 79.3 | 501.3 |
| | 300 | 49.0 | 92.5 | 748.1 |
| Comparative catalyst 2 | 220 | 21.6 | 80.3 | 286.3 |
| | 240 | 37.5 | 84.9 | 525.8 |
| | 260 | 65.3 | 96.4 | 1038.4 |
| | 280 | 74.5 | 98.2 | 1207.4 |
| | 300 | 64.5 | 95.5 | 1016.0 |
| 1 | 220 | 17.3 | 58.5 | 169.6 |
| | 240 | 30.8 | 81.6 | 421.0 |
| | 260 | 62 | 96.0 | 999.2 |
| | 280 | 70.2 | 97.3 | 1147.6 |
| | 300 | 64.3 | 95.3 | 1029.1 |
| 2 | 220 | 24.0 | 70.6 | 279.7 |
| | 240 | 44.0 | 90.4 | 656.8 |
| | 260 | 68.7 | 97.1 | 1101.1 |
| | 280 | 74.5 | 97.9 | 1203.9 |
| | 300 | 64.9 | 95.6 | 1023.9 |
| 3 | 220 | 23.0 | 76.1 | 363.2 |
| | 240 | 42.9 | 89 | 629.8 |
| | 260 | 67.7 | 97.8 | 1093..5 |
| | 280 | 73.7 | 97.8 | 1189.8 |
| | 300 | 64.3 | 95.5 | 1013.6 |
| 3' | 220 | 19.8 | 72.0 | 235.3 |
| | 240 | 42.9 | 87.5 | 619.6 |
| | 260 | 67.7 | 98.3 | 1098.5 |
| | 280 | 73.7 | 98.4 | 1197.1 |
| | 300 | 63.5 | 97.8 | 1025.1 |
| 4 | 220 | 22.6 | 73.3 | 273.2 |
| | 240 | 36.7 | 88.0 | 532.4 |
| | 260 | 61.2 | 96.0 | 970.2 |
| | 280 | 68.3 | 86.8 | 1092.3 |
| | 300 | 64.2 | 95.1 | 1008.3 |
| 4' | 220 | 24.4 | 75.5 | 304.0 |
| | 240 | 39.8 | 88.7 | 583.0 |
| | 260 | 66.6 | 97.0 | 1066.3 |
| | 280 | 72.4 | 97.5 | 1166.0 |
| | 300 | 64.8 | 95.6 | 1022.1 |
| 5 | 220 | 17.3 | 60.3 | 172.2 |
| | 240 | 36.4 | 84.1 | 505.3 |
| | 260 | 65.7 | 96.9 | 1050.0 |
| | 280 | 72.5 | 97.8 | 1170.5 |
| | 300 | 64.4 | 95.6 | 1016.5 |
| 5' | 220 | 24.1 | 70.8 | 282.2 |
| | 240 | 41.3 | 88.6 | 603.9 |
| | 260 | 65.2 | 96.7 | 1040.5 |
| | 280 | 71.2 | 97.5 | 1146.3 |
| | 300 | 64.5 | 95.2 | 1013.4 |

### CITATION LIST

1. Hutchings G.J., Védrine J.C. (2004) Heterogeneous Catalyst Preparation. In: Baerns M. (eds) Basic Principles in Applied Catalysis. Springer Series in Chemical Physics, vol 75. Springer, Berlin, Heidelberg
2. Atake et al., Journal of Molecular Catalysis A: Chemical 275 (2007) 130-138.
3. Fan et al. Fuel Processing Technology 167 (2017) 69-77.
4. Behrens et al. Chem. Commun., 2011, 47, 1701-1703.
5. Prieto et al. Catalysis Today 215 (2013) 142- 151.
6. Smith et al. Chem. Sci., 2017, 8, 2436.
7. Bansode et al. Journal of Catalysis 343 (2016) 127-132.

## Claims

1. Method for the preparation of a catalyst precursor comprising the steps of:
(i) heating an aqueous solution of urea with a mixture of metal salts comprising at least a compound of formula Cu(OCOCH₃)₂, a solvate thereof or a co-crystal thereof, to obtain a solid, wherein the molar amount of urea is at least three times the total molar amount of metal salts;
(ii) separating the solid obtained in step (i) from the aqueous solution; and
(iii) calcinating the solid obtained in step (ii).

2. Method in accordance with claim 1 wherein the mixture of metal salts of step (i) comprises salts of metal cations with anions other than nitrate.

3. Method in accordance with any one of claims 1 or 2, wherein the mixture of metal salts used in step (i) further comprises a compound of formula Zn(OCOCH₃)₂, a solvate thereof or a co-crystal thereof.

4. Method in accordance with any one of claims 1 to 3, wherein the mixture of metal salts comprises substantially Cu(OCOCH₃)₂, a solvate thereof or a co-crystal thereof and Zn(OCOCH₃)₂, a solvate thereof or a co-crystal thereof; preferably wherein the mixture of metal salts consists in a mixture of Cu(OCOCH₃)₂, a solvate thereof or a co-crystal thereof and Zn(OCOCH₃)₂, a solvate thereof or a co-crystal thereof.

5. Method in accordance with claim 3 to 6, wherein the molar ratio of Cu(OCOCH₃)₂ to Zn(OCOCH₃)₂ in the mixture of metal salts is between 3:1 and 1:3; preferably the molar ratio of Cu(OCOCH₃)₂ to Zn(OCOCH₃)₂ is between 2:1 to 1:2; and more preferably the molar ratio of Cu(OCOCH₃)₂ to Zn(OCOCH₃)₂ is 1:1.

6. Method in accordance with any one of claims 1 to 5, wherein step (i) is carried out at a temperature between 70 °C and 90 °C, preferably of about 80 °C.

7. Method in accordance with any one of claims 1 to 6, wherein the molar amount of urea used in step (i) is between 3 and 10 times the total molar amount of the mixture of metal salts.

8. Method in accordance with any one of claims 1 to 7, wherein step (i) is carried out during a time of between 12 and 24 hours.

9. The method in accordance with any one of claims 1 to 8 consisting of the steps (i), (ii) and (iii).

10. Catalyst precursor obtained by means of a method according to any one of claims 1 to 9.

11. Method for the preparation of a catalyst comprising the step of activating the catalyst precursor in accordance with claim 10.

12. Catalyst obtained by means of a method in accordance with claim 11.

13. Composition comprising a catalyst precursor in accordance with claim 10 or; a catalyst in accordance with claim 12.

14. Use of a catalyst in accordance with claim 12 or a catalyst composition in accordance with claim 13 as a catalyst in a method for the preparation of methanol by hydrogenation of carbon dioxide or for the low temperature water gas shift reaction.

15. Method for the preparation of methanol by hydrogenation of carbon dioxide comprising the step of contacting carbon dioxide with hydrogen in the presence of a catalytically effective amount of a catalyst in accordance with claim 12 or a catalyst composition in accordance with claim 13, preferably wherein the pressure is equal to or higher than 150 bar; the temperature is between 200 and 330 °C; and the space velocity is at least 500 h⁻¹.
